# FASCICULE DE BREVET EUROPEEN

(11) **EP 0 906 044 B1**
(45) Date de publication et mention de la délivrance du brevet: **21.11.2001**
(21) Numéro de dépôt: 97917378.8
(22) Date de dépôt: 05.05.1997
(51) Int. Cl.: A47C 21/00, A47G 9/00

(54) **BIO-COIFFE ANATOMIQUE ANTIRIDES FACIALES ET SA TAIE**
ANATOMISCHE KAPPE ZUR VERHÜTUNG VON GESICHTSFALTEN SOWIE BEZUG
ANATOMIC ANTI-WRINKLE BIO-CAP AND ITS CASE

(30) Priorité: 22.05.1996 FR 9606333
(43) Date de publication de la demande: 07.04.1999
(73) Titulaire: Ionescu, Raoul Michel, 78175 Saint Germain en Laye Cedex (FR)
(72) Inventeur: Ionescu, Raoul Michel, 78175 Saint Germain en Laye Cedex (FR)
(86) Numéro de dépôt international: IB9700484
(87) Numéro de publication internationale: WO9743926

(56) Documents cités:
- DE-C- 294 917
- FR-A- 619 587
- US-A- 5 220 699
- US-A- 5 269 035

## Description

La présente invention concerne une BIO-COIFFE ANATOMIQUE destinée à être utilisée, comme montré en figures (4), (5), (6), (7), (8), (9) et (10), à la place des oreillers traditionnels et/ou traversins traditionnels et/ou tout autre objet utilisé couramment et spécialement par une personne physique pendant son sommeil et/ou son repos pour poser et/ou appuyer sa tête, objets qui seront globalement dénommés ci-après : OREILLERS ET/OU TRAVERSINS.
Le document de brevet allemand DE-C- 294917 du 19 nov. 1915 décrit un coussin de repos sous la forme d'une coiffe ayant une partie principale (couvrant l'arrière de la tête et les oreilles de la personne) et deux parties rabattables attachées (couvrant les yeux - si déployées), pour isoler contre tout bruit et lumière. Ce document ne donne aucune indication quant aux épaisseurs des differents parties du coussin.
L'utilisation de la BIO-COIFFE ANATOMIQUE à la place des oreillers et/ou traversins, crée pour la personne physique qui l'utilise un cadre-environnement générateur d'apports biologiquement bénéfiques pour l'organisme de la dite personne - d'où le préfixe BIO - en éliminant tout une série d'inconvénients liés à l'usage des oreillers et/ou traversins pendant le sommeil et/ou le repos de la dite personne en position allongé/couché, par exemple, sur un lit.
Parmi les inconvénients ci-dessus évoqués, majoritairement causés par les contacts et pressions qui s'exercent entre la peau du visage de l'utilisateur et les oreillers et/ou traversins, voici une énumération non exhaustive :
- la formation des plis de la peau du visage qui, à la longue, se transforment en rides permanentes ;
- mauvaise irrigation sanguine au niveau des capillaires, qui accélère le vieillissement de la peau ;
- obturation d'une narine - pour les positions sur les côtés - ce qui empêche une respiration fluide avec comme conséquence une oxygénation diminuée de l'organisme pendant le sommeil ;
- obturation possible des deux narines - pour la position sur le ventre - avec comme conséquence une suffocation dangereuse, surtout pour les enfants ;
- pressions non naturelles sur les globes oculaires - pour la position sur le ventre ;
- non respect du trajet cervicales, axis, atlas, trou occipital ;
- favorisation du ronflement, pour la position sur le dos.

Chaque position de la personne utilisant oreillers et/ou traversins pendant son sommeil, comporte ses inconvénients spécifiques.
Un trait essentiel de ces inconvénients est que les effets de leurs nuisances sur l'organisme ont une progression selon une allure géométrique (et non linéaire) par rapport à leurs durées, égales en principe avec le temps pendant lequel la personne ne change pas de position. Par exemple, une mauvaise respiration provoque pendant sa 2-ème minute plus de dégâts que pendant sa 1-ère minute et moins qu'elle va provoquer pendant sa 3-ème minute et ainsi de suite. La durée totale d'une position peut varier, de quelques minutes jusqu'à une heure, même plus.

Ci-après, une analyse détaillée de chaque cas.

Tout le long d'un pli de la peau du visage, pli formé par contact contre les oreillers et/ou traversins, la circulation sanguine au niveau des capillaires est pratiquement bloquée et les tissus ne sont plus normalement irrigués. Plus cette situation dure, plus les dégâts sont irréversibles. Or, la durée est assez longue et fréquemment répétée car favorisée par les habitudes particulières de la personne pendant le sommeil : positions et attitudes préférées ce qui fait que les plis se répètent aux mêmes endroits et qu'ils durent longtemps : conditions réunies pour qu'ils se transforment en rides permanentes. Cela conduit au fait que ces rides varient - mais pas trop - d'une personne à l'autre. Et plus la peau du visage est vieillie, plus ces rides s'installent dans un laps de temps plus court.
Les premières rides qui apparaissent - et pratiquement pour toutes les personnes, dès l'âge jeune - sont les deux rides qui encadrent le nez à sa base et descendent dans un premier temps, obliquement vers les extrémités des lèvres. Elles se forment en dormant sur les côtés, oreille sur l'oreiller : la tête s'enfonce dans l'oreiller et la peau de la joue, retenue par le contact contre l'oreiller, glisse vers le nez dans une tendance à l'envelopper. Pire encore : en dormant sur le ventre avec la tête tournée vers la gauche c'est la ride de droite (de la personne) qui se creuse (et avec la tête tournée à droite c'est celle de gauche) ; dans ces positions, la joue entière est translatée vers le nez qui y fait obstacle ce qui rend le pli assez brutal. Cette ride se caractérise par le fait qu'elle descend, avec l'âge et pour la plupart des personnes, jusqu'au bas du menton. Ce n'est qu'un exemple. En général, les rides causées par oreillers et/ou traversins sont des rides verticales et/ou légèrement obliques sur le visage. Mais elles sont très profondes et très longues.

Mis à part les plis, les pressions qui s'exercent sur de grandes parties de la peau du visage qui viennent en contact avec les oreillers et/ou traversins, rendent plus difficile l'irrigation sanguine capillaire au niveau du derme de la surface en contact, en accélérant ainsi le vieillissement de la peau ce qui va faciliter, entre autres, l'installation de rides de tout sorte.

L'obturation d'une narine pour la position sur le côté ou sur le ventre, conduit à une moindre fluidité de la respiration et donc à une oxygénation diminuée de l'organisme pendant le sommeil. Les conséquences négatives sur l'organisme entier de ce manque d'oxygénation sont longues à énumérer car cela concerne pratiquement la majorité des fonctions physiques et psychiques de l'organisme qui, en plus, s'imbriquent ce qui conduit au fait que la récupération générale, au moment de l'éveil, n'est que partielle par rapport à la durée totale du sommeil.

L'obturation des deux narines - possible pour la position sur le ventre - peut provoquer une suffocation qui est très dangereuse, surtout pour les enfants car elle peut-être totale, l'ossature du nez n'étant pas encore totalement accomplie et les reflexes contre une telle suffocation pas encore acquis. Cette suffocation qui peut dépasser même une minute avant que la personne ne réagisse machinalement, ne fait qu'aggraver ce qui vient d'être dit sur la non oxygénation de l'organisme avec la précision que dans ce cas c'est le cerveau qui sera malmené car il supporte mal les pauses dans son irrigation avec du sang oxygéné : au-delà de quelques minutes, les dégâts risquent d'être irréversibles.

Les pressions sur les globes oculaires qui ont lieu pour les positions sur le ventre et celles proches de cette position, déplacent les globes par rapport à leurs positions normales, vers l'intérieur du crâne avec des conséquences qui probablement n'ont pas été suffisamment analysées. L'inventeur n'a trouvé nulle part une étude en ce sens. Deux choses viennent normalement à l'esprit. Une première est que ce déplacement des globes modifie la position normale des petits vaisseaux sanguins qui les irriguent, avec des posibilités de strangulation partielle ou non. Une deuxième est que ces pressions extérieures sur les globes, vu la résistance en sens contraire qui est opposée, vont inéluctablement conduire à l'augmentation de la pression à l'intérieur des globes pour des longues durées (tant que la position sur le ventre est maintenue). Or, il est établi que l'augmentation au-delà de la normale de la pression à l'intérieur du globe oculaire (appelée couramment tension oculaire) est un signe précurseur du glaucome, grave maladie de l'oeil. En l'absence d'une étude précise sur ces aspects, l'inventeur se résumera à conclure que, apparemment, mieux vaut éviter ces pressions prolongées et non naturelles sur les globes oculaires.

Le trajet cervicales, axis, atlas, trou occipital est difficile à respecter dans le cas des oreillers et/ou traversins car ces derniers sont pratiquement fixes et les positions de l'utilisateur très variées et totalement imprévisibles. Il en résulte un non respect graduel de ce trajet pour une partie de la durée du sommeil, difficile à évaluer car elle dépend des habitudes particulières à chaque personne pendant le sommeil. Le non respect du dit trajet se traduit par des maux qui empiètent sur l'état général du sommeil et donc sur celui de la personne au moment du réveil.

Les oreillers et/ou traversins ne provoquent pas eux-mêmes le ronflement ou les causes de celui-ci, mais ils contribuent à l'amplifier,par exemple pour la position sur le dos et un oreiller trop haut. Et ils n'ont aucune fonction qui pourrait le faire baisser ou le prévenir. Les balles de tennis et autres objets fixé(e)s au dos des pyjamas peuvent aider à éviter la position sur le dos mais, il faut avoir à l'esprit le fait que c'est plutôt la position de la tête face au corps qu'il faut considérer. Les conséquences négatives du ronflement sur l'organisme sont de la même nature que celles dues aux obturations des narines avec en plus, le bruit désagréable pour la personne elle-même et surtout pour l'entourage.

Tous ces inconvénients avec leurs multiples et très variées conséquences négatives sur l'efficacité de l'action réparatrice pendant la période du sommeil, doivent être pris en considération attentivement car il s'agit d'une très longue période totale qui pratiquement s'étend sur un tiers de la vie entière. Si on réussissait à améliorer la qualité du sommeil, il sera peut-être possible d'écourter sa durée en gagnant ainsi sur la durée de la vie en état d'éveil qui elle est effective.

L'utilisation comme montré dans les figures (4), (5), (6), (7), (8), (9) et (10) de la BIO-COIFFE ANATOMIQUE par une personne physique, à la place des oreillers et/ou traversins, élimine pratiquement tous les inconvénients de ceux-ci, ci-dessus détaillés, car elle élimine toute possibilité de contact du visage de la dite personne, avec tout autre objet pouvant entrainer des contacts et/ou pressions nuisibles. Il en résulte, et cela pour n'importe quelle position de la personne qui utilise la BIO-COIFFE ANATOMIQUE :
- impossibilité de formation des plis de la peau du visage par contacts et pressions, donc les rides afférentes à ces plis ne peuvent plus se creuser ; mieux encore, les rides qui sont déjà en place auront tendance à s'effacer sous le mécanisme propre aux tissus organiques de se régénérer en l'absence prolongée de la nuisance ; si les rides déjà en place ne sont pas trop creusées, elles pourront même s'effacer totalement ;
- la peau du visage entier sera, pendant toute la durée du sommeil, irriguée normalement car aucune pression extérieure ne viendrait s'exercer contre elle ; les divers crèmes que la personne utilisatrice aurait appliquées sur les joues et le cou resteront en place et ne seront pas effacées par contact avec oreillers et/ou traversins ; la pression exercée sur le front par la BIO-COIFFE ANATOMIQUE elle-même est très faible car uniformement repartie et effective seulement en position sur le ventre, assez rare ; comme on le verra plus loin, la taie propre à la BIO-COIFFE ANATOMIQUE, empêche elle-même la formation de rides sur le front et contribue efficacement à l'élimination de celles déjà en place avant l'utilisation de la BIO-COIFFE ANATOMIQUE ;
- aucune possibilité d'obturation de(s) narine(s) ;
- aucune pression sur les globes oculaires ;
- respect automatique pendant toute la durée du sommeil du trajet cervicales, axis, atlas, trou occipital car la BIO-COIFFE ANATOMIQUE est solidaire de la tête de la personne qui l'utilise ;
- quant au ronflement, si la BIO-COIFFE ANATOMIQUE ne peut pas le guérir, elle à la capacité de signaler, discrètement et seulement à l'oreille de la personne qui l'utilise, sa position sur le dos (qui favorise le ronflement) pour qu'elle la change en une autre moins propice au ronflement ; l'arrêt de la signalisation se produit automatiquement dès que la personne a quitté la position sur le dos ; en plus, une barrette (R) sur les figures (4) et (7), est prévue pour être placée en légère tension élastique (réglable) sur le menton de la personne utilisatrice, ce qui fait que la personne ne pourra plus dormir avec les dents serrées ce qui, dans beaucoup de cas, élimine le ronflement ou tout au moins le réduit sensiblement ;
- la BIO-COIFFE ANATOMIQUE permet en plus une isolaton phonique réglable comme efficacité, contre les bruits environnants.

Tous ces apports de la BIO-COIFFE ANATOMIQUE sont rendus possibles par son attribut essentiel, constitué par sa forme fonctionnelle conjugué au fait que la BIO-COIFFE ANATOMIQUE est solidaire de la tête de la personne qui l'utilise ce qui conduit à éliminer tout possibilité de contact du visage de la dite personne avec tout objet pouvant entrainer des pressions nuisibles comme dans le cas des oreillers et/ou traversins et cela sans aucun arceau rigide visible, incommodant et inesthétique, devant le visage de la personne qui l'utilise pendant son sommeil et/ou son repos, allongée/couchée dans n'importe quelle position - sur le ventre, sur le dos, sur les côtés, dans des positions intermédiaires à celles énumérées, recroquevillées - sur une surface plane et complètement dégagée de tout objet, y compris oreillers et/ou traversins, comme celle d'un lit pour une personne ou celle destinée à une personne en cas d'un grand lit pour deux personnes, lits horizontaux et normaux, munis ou non munis de mécanismes actionnés ou non actionnés pour faire incliner à des angles habituels la partie où on pose la tête et/ou la partie où on pose les pieds et/ou toutes autres régions transversales de leurs surfaces, lits couverts ou non couverts d'un matelas et avec/sans drap.

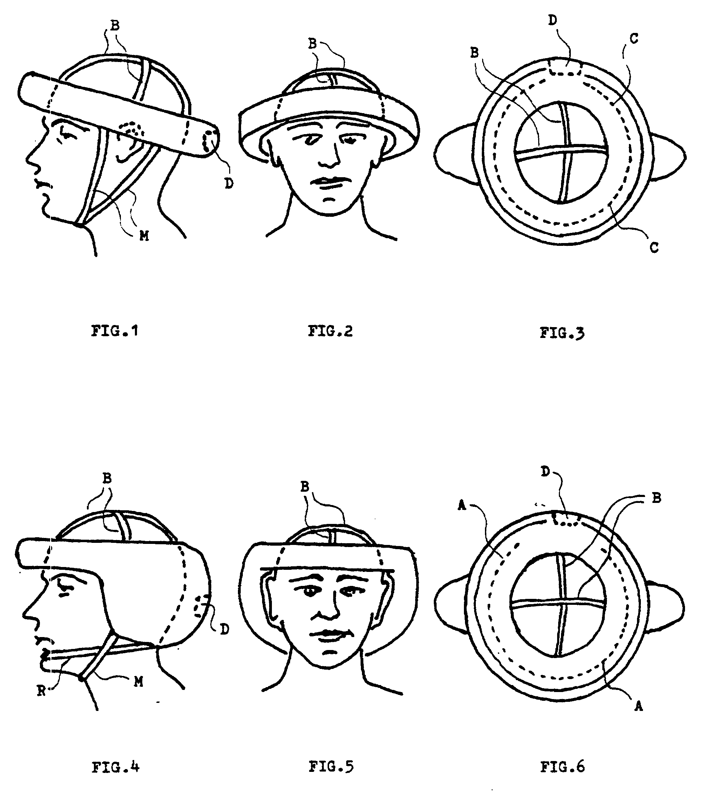

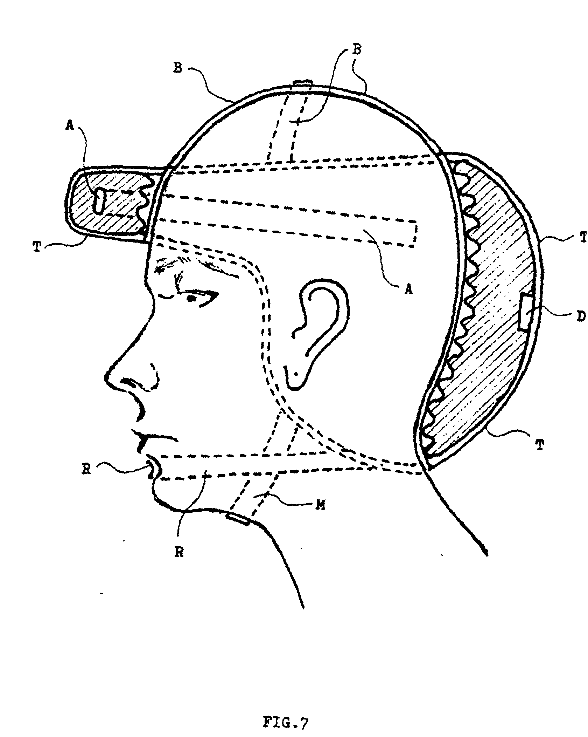

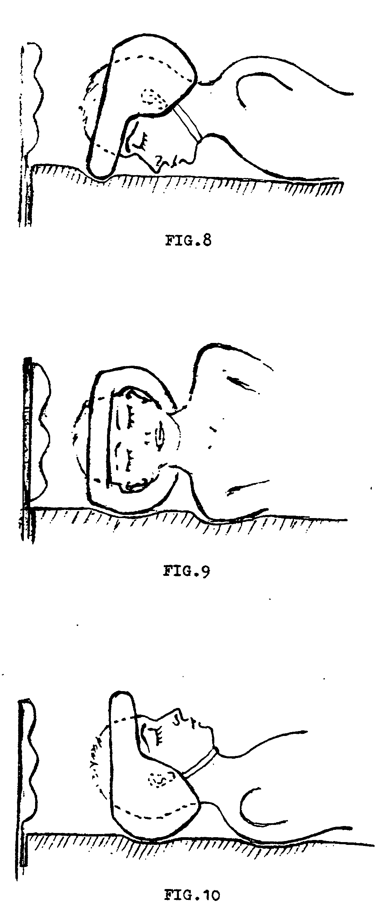

Les figures (4), (5) et (6) présentent la BIO-COIFFE ANATOMIQUE dans sa forme la plus accomplie, qui couvre en totalité les oreilles et la convexité du derrière du crâne, et sa manière d'être portée par une personne qui est représentée debout pour faciliter les explications qui suivent. Les figures (8), (9) et (10) représentent une personne utilisant la BIO-COIFFE ANATOMIQUE en position allongé/couché sur un lit. La figure (7) représente une section de la BIO-COIFFE ANATOMIQUE dans la situation présentée par la figure (4), selon un plan imaginaire défini comme équidistant des yeux, des narines et des oreilles de la personne qui porte la BIO-COIFFE ANATOMIQUE. Pour la clarté du dessin, les cheveux de la dite personne, n'ont pas été représentés.

Les figures (1), (2) et (3) représentent la BIO-COIFFE ANATOMIQUE dans sa forme la plus réduite, ressemblant à celle d'une sorte de turban, mais qui néanmoins, assure la totalité de ses fonctions ci-haut décrites avec la mention qu'elle procure un moindre respect du trajet cervicales, axis, atlas, trou occipital que la forme représentée dans toutes les autres figures. La forme réduite présentée en figures (1), (2) et (3) a l'avantage d'une fabrication plus facile, comme ci-après décrit. La BIO-COIFFE ANATOMIQUE peut prendre n'importe quelle forme intermédiaire entre celle présentée en figures (1), (2) et (3) et la forme la plus accomplie des figures (4) à (10), tout en assurant la totalité des fonctions ci-haut décrites avec évidemment la mention que te respect du trajet cervicales, axis, atlas, trou occipital sera graduellement amélioré au fur et à mesure qu'on se rapproche de sa forme la plus accomplie présentée en figures (4) à (10) couvrant en totalité les oreilles et la convexité du derrière du crâne de l'utilisateur.

Dans tous les cas, des cavités appropriées seront prévues à l'intérieur de la BIO-COIFFE ANATOMIQUE pour que les oreilles de la personne qui l'utilise puissent se loger sans que des pressions s'exercent sur elles en position debout, non allongé. Ces cavités seront prolongées vers l'avant, pour la forme présentée en figures (4), (5), (6), (7), (8), (9) et (10) de la sorte que les sons extérieurs puissent être normalement entendus.

Un emplacement (D) sur les figures (1), (3), (4), (6) et (7), est prévu pour un détecteur de la position sur le dos, position qui favorise le ronflement. Il est constitué par un contact électrique qui s'établit suite au poids de la tête de la personne, qui presse contre le matelas pendant la position sur le dos ou proche de cette position. Le contact établit provoque l'alimentation, par une petite pile de 1,5 volts, d'un circuit électronique à base de transistors qui génère un courant alternatif de basse fréquence d'env. 800 cycles par seconde qui est envoyé sous forme d'impulsions répétées environ 2 fois par seconde vers deux haut-parleurs miniaturisés placés dans la BIO-COIFFE ANATOMIQUE, chacun devant l'une des oreilles de la personne. La proximité du haut-parleur de l'oreille de la personne permet que les sons ne soient pas trop forts pour déranger une autre personne se trouvant dans le voisinage ; cette condition est moins remplie par une BIO-COIFFE ANATOMIQUE de la forme présentée en figures (1), (2) et (3). Le contact s'ouvre coupant l'alimentation par la pile électrique, avec l'arrêt des sons émis, dès que la personne a quitté sa position sur le dos. L'enlèvement de la pile électrique arrête le fonctionnement du dit détecteur. Les circuits électroniques générateurs de la fréquence basse sous forme d'impulsions répétées, pouvant prendre une grande variété de réalisations pratiques, ne font pas l'objet du présent brevet.

La BIO-COIFFE ANATOMIQUE sera confectionnée dans des matériaux non rigides et élastiques comme, par exemple, la mousse de polyuréthane ou similaires, les éponges spongiaires, l'air retenu dans une enveloppe étanche de la forme de la BIO-COIFFE ANATOMIQUE et pourvue d'une valve pour permettre son gonflage et son dégonflage. Des mesures appropriées seront prises pour la BIO-COIFFE ANATOMIQUE gonflable pour qu'elle prenne la forme correcte après gonflage comme des renforts et ligaments intérieurs dans les endroits appropriés. Pour le cas de la mousse polyuréthane et similaires, des ondulations de forme conique avec sommets arrondis seront prévues pour toute partie de la BIO-COIFFE ANATOMIQUE qui avoisine de près la peau de la personne qui l'utilise, comme indiqué en figure (7). Pour la partie qui couvre le front et les tempes de la personne, un arceau (A) sur la figure (7), en matériel non deformable plastiquement et élastique - de préférence en plastique,ébonite - sera encastré comme montré en figures (7) et (6) sur une longueur d'environ 270° de la circonférence de la BIO-COIFFE ANATOMIQUE et centré sur le milieu du front de la personne utilisatrice, pour assurer la stabilité de la partie de la BIO-COIFFE ANATOMIQUE qui couvre le front et les tempes, par rapport au reste de la BIO-COIFFE ANATOMIQUE. Cet arceau sera étendu pour la forme simplifiée de la BIO-COIFFE ANATOMIQUE représentée en figures (1), (2) et (3) sur toute la circonférence, moins l'espace destiné au détecteur (D). Ce cas est représenté par (C) sur la figure (3).

Comme la BIO-COIFFE ANATOMIQUE est destinée à être utilisée par tout le monde de l'enfant jusqu'à l'adulte, femmes et hommes, elle sera fabriquée dans des tailles standardisées, comme les habits, les chaussures ; ou mieux, sur mesure. Il n'est donc pas possible de donner des dimensions précises pour sa réalisation mais, à titre d'exemple, indiquons quelques ordres de grandeur pour un homme adulte de taille moyenne :
- épaisseur de la partie frontale pour assurer le dégagement du nez en position sur le ventre : environ 7 cm.
- épaisseur de la partie correspondante à l'endroit de l'oreille pour assurer en position sur les côtés, le respect du trajet cervicales, axis, atlas, trou occipital : environ 8 cm.
- épaisseur dans la partie du centre de la convexité, derrière du crâne, à l'endroit destiné pour le détecteur de position sur le dos (D) sur la figure (7), pour assurer le respect du trajet cervicales axis, atlas, trou occipital en position sur le dos : environ 7 cm.
- hauteur de la partie frontale et tempes : environ 6 cm.

Les épaisseurs entre ces parties seront celles qui en résultent par raccordements graduels de telle sorte que l'aspect général de la BIO-COIFFE ANATOMIQUE apparaît comme pratiquement rond.

Les dimensions citées sont informatives car pour bien assurer le respect du trajet cervicales, axis, atlas, trou occipital il faut tenir compte du reste de la conformation de la personne qui va utiliser la BIO-COIFFE ANATOMIQUE, comme la largeur des épaules et la denivelation moyenne de la convexité de la courbure du dos de la personne et c'est pour cette raison que, pour des cas particuliers, mieux vaut executer la BIO-COIFFE ANATOMIQUE sur mesure (ce qui se passe aussi pour les habits, chaussures) d'autant plus que les moyens actuels de fabrication, comme on le verra plus loin, permettent de façonner la BIO-COIFFE ANATOMIQUE personnalisée d'une manière très précise et dans un laps de temps très court.

La BIO-COIFFE ANATOMIQUE sera utilisée dans une enveloppe-taie, de tissu lavable, remplissant le rôle d'une taie habituelle d'oreiller. Cette enveloppe-taie, qu'on va dénommer ci-après TAIE, est désignée sur la figure (7) par (T). Elle aura la même forme que celle délimitée par la superficie totale (extérieure et intérieure) de la BIO-COIFFE ANATOMIQUE, forme qui tiendra compte des tolérances nécessaires pour qu'elle puisse envelopper/habiller la BIO-COIFFE ANATOMIQUE. Cette taie se refermera comme une taie habituelle d'oreiller, par des boutons traditionnels, boutons pression, fermeture éclair, placé(e)s de telle manière pour ne pas gêner la personne qui utilise la BIO-COIFFE ANATOMIQUE, par exemple dans la partie correspondante au dessus de la BIO-COIFFE ANATOMIQUE. Cette taie servira aussi de support pour les points d'ancrage pour la mentonnière (M) sur les figures annexées, les bandelettes (B) sur les figures annexées, la barrette anti-ronflement (R) sur les figures annexées. L'ancrage sera ou définitif ou détachable à l'aide de boutons pression par exemple, pour pouvoir enlever mentonnières et/ou bandelettes et/ou barrette anti-ronflement le cas échéant. De même, pour les formes de BIO-COIFFES ANATOMIQUES qui couvrent complètement les oreilles, des points d'attache seront prévus à l'intérieur des cavités pour les oreilles pour pouvoir y fixer des objets flexibles en matières qui absorbent les sons, pour créer une isolation de la personne face aux bruits environnants, le cas échéant.

La partie de la taie qui vient en contact avec le front sera prévue d'une certaine élasticité, par exemple par l'inclusion de fibres élastiques, élasticité s'exerçant parallèlement avec le front de la personne utilisatrice, afin d'assurer un contact permanent faible et contrôlé avec la peau du front pour empêcher la formation des plis qui risqueront de se transformer en rides permanentes. Cette pression faible, uniformement répartie et constante contribue elle même à l'effacement des rides creusées avant l'utilisation de la BIO-COIFFE ANATOMIQUE, si elles ne sont déjà trop profondes.

En regardant la figure (7) on remarque que la BIO-COIFFE ANATOMIQUE épouse la forme de la tête de la personne qui l'utilise - d'où l'adjectif ANATOMIQUE - et qu'elle peut rester en place suite à la courbure présentée par la partie derrière du crâne. Toutefois, dans certains cas, par exemple pour les personnes qui ont un sommeil agité, il existe le risque que la BIO-COIFFE ANATOMIQUE quitte sa position correcte sur la tête, surtout pour sa forme réduite représentée sur les figures (1), (2) et (3). Pour cette raison des bandelettes (B) sur les figures annexées, ont été prévues pour la partie haute du crâne et une mentonnière, double pour le modèle réduit de la figure (1), pour assurer le maintien en position correcte de la BIO-COIFFE ANATOMIQUE. Les bandelettes et les mentonnières seront executées en tissus lavables non extensibles avec, le cas échéant, possibilité de régler leur longueur comme pour les ceintures par exemple pour pouvoir bien ajuster et assurer la stabilité de la BIO-COIFFE ANATOMIQUE en position sur la tête. La barette anti-ronflement sera aussi réglable en longueur mais une partie de sa longueur, respectivement un tiers de son trajet qui reste en permanence en contact avec la peau de l'utilisateur, sera en matériel élastique ce qui donne la possibilité de régler la tension vers le bas qu'elle va exercer sur le menton de l'utilisateur, en réglant sa longueur. Le rôle de cette barette est d'empêcher la personne de dormir avec les dents serrées, car cela favorise le ronflement. Dans beaucoup de cas, le fait de desserrer les dents jusqu'à la limite quand les lèvres restent encore non séparées, élimine le ronflement ou le réduit.

La BIO-COIFFE ANATOMIQUE peut être fabriquée soit artisanalement, soit d'une manière industrielle qui elle peut être poussée à une productivité extrême par automatisation, après l'établissement d'une échelle de tailles standardisées, comme pour les chaussures par exemple. La manière artisanale consisterait dans le façonnage de la forme de la BIO-COIFFE ANATOMIQUE, selon les mensurations d'une personne, dans un bloc de mousse de polyuréthane de dimensions suffisantes et puis de manufacturer la taie, les bandelettes, mentonnières, barrettes anti-ronflement, ce qui ne poserait aucun problème particulier car ce sont des techniques couramment connues et pratiquées. L'inconvénient de la manière artisanale réside dans sa productivité réduite et son avantage, dans le fait qu'elle permet une confection sur mesure.

La manière industrielle permet de réaliser une grande productivité et grâce aux possibilités de l'informatique de réaliser aussi, facilement, du sur mesure. Il n'y a aucune partie constituante de la BIO-COIFFE ANATOMIQUE qui puisse poser des problèmes de fabrication non connus à ce- jour ou à développer en l'occurrence, pour sa fabrication en très grande série. Je vais détailler ci-après le cas du sur mesure par voie industrielle.

Les mensurations nécessaires pour établir la forme exacte de la BIO-COIFFE ANATOMIQUE qui corresponde à une personne particulière, seront prises par exemple dans un point de vente de BIO-COIFFES ANATOMIQUES, par une personne qui va les introduire, via un clavier, dans un ordinateur qui contient un logiciel spécialement conçu et adapté à cette fin. Après avoir pris toutes les mesures nécessaires et que le client ait choisi diverses options comme la couleur, le matériel, le dessein de la taie, avec ou sans avertisseur sonore etc., l'ordinateur traite ces informations et établit une liste de données qu'il va transmettre lui même à l'usine productrice, données qui serviront à programmer d'une manière adéquate les paramètres de la ligne de fabrication pour qu'elle puisse réaliser dans les plus brefs délais la BIO-COIFFE ANATOMIQUE commandée et sa taie qui pourront être après, incessamment envoyées directement au domicile de l'acheteur.

Un soin particulier apporté à l'exécution et à la présentation de la taie, avec des motifs variés comme forme et coloris, aura comme résultat de lui rendre un aspect esthétique particulier et attractif.

L'utilisation de la BIO-COIFFE ANATOMIQUE par des personnes ne pose pas de problèmes particuliers. Après quelques jours d'utilisation et une fois dissipée la retenue qu'on ressent initialement face à un objet qui ne ressemble guère à un oreiller ou à un traversin traditionnel et surtout, après s'être convaincu personnellement de ses apports biologiquement bénéfiques, la routine de son utilisation est pratiquement déjà en place. La BIO-COIFFE ANATOMIQUE s'enlève d'un mouvement de la main, après avoir défait, le cas échéant, sa mentonnière.

La BIO-COIFFE ANATOMIQUE, objet de cette invention, est destinée à tout le monde.

## Revendications

1. Bio-coiffe anatomique, objet destiné à un usage courant à la place d'un oreiller et/ou d'un traversin par les personnes physiques, épousant et étant solidaire de la tête de son utilisateur pendant son sommeil et/ou son repos en position couché/allongé, bio-coiffe anatomique comportant :
- une partie frontale,
- deux parties correspondant aux endroits des oreilles et
- une partie correspondant au centre de la convexité du derrière du crâne,
bio-coiffe anatomique **caractérisée en ce que**, tenant compte des conformations physiques propres à l'utilisateur,
- l'épaisseur de la bio-coiffe anatomique dans la partie frontale est telle que, lorsque l'utilisateur est en position couché/allongé sur le ventre, la bio-coiffe anatomique assure le dégagement du nez de l'utilisateur,
- les épaisseurs de la bio-coiffe anatomique dans les parties correspondant aux endroits des oreilles sont telles que, lorsque l'utilisateur est en position couché/allongé sur les côtés, la bio-coiffe anatomique assure le respect du trajet cervicales, axis, atlas, trou occipital de l'utilisateur,
- l'épaisseur de la bio-coiffe anatomique dans la partie correspondant au centre de la convexité du derrière du crâne est telle que, lorsque l'utilisateur est en position couché/ allongé sur le dos, la bio-coiffe anatomique assure le respect du trajet cervicales, axis, atlas, trou occipital de l'utilisateur,
- lesdites parties étant raccordées entre elles, les raccordements concernant leurs épaisseurs se faisant graduellement pour parachever le contour fermé de la bio-coiffe anatomique, pratiquement rond, autour de la tête de l'utilisateur,
grâce à quoi, la bio-coiffe anatomique assure aussi :
le respect du trajet cervicales, axis, atlas, trou occipital de l'utilisateur, lorsqu'il se trouve dans des positions couché/allongé intermédiaires à celles citées et simultanément, l'élimination de toute possibilité de contact du visage de l'utilisateur avec tout autre objet pouvant entrainer des pressions nuisibles, quelque soit la position couché/allongé de l'utilisateur sur une surface dégagée de tout objet, pendant son sommeil et/ou son repos.

2. Bio-coiffe anatomique selon la revendication 1, **caractérisée en ce que** la bio-coiffe anatomique peut prendre n'importe quelle forme entre une forme réduite analogue à un turban et une forme accomplie couvrant en totalité les oreilles et la convexité du derrière du crâne.

3. Bio-coiffe anatomique selon Tune quelconque des revendications 1 ou 2, **caractérisée en ce qu'**elle est fabriquée en un matériau non-rigide et élastique.

4. Bio-coiffe anatomique selon l'une quelconque des revendications 1 ou 2, **caractérisée en ce qu'**elle est constituée par une enveloppe étanche gonflable à l'air.

5. Bio-coiffe anatomique selon la revendication 3, **caractérisée en ce que** le matériau est une mousse de polyuréthane ou une éponge spongiaire.

6. Bio-coiffe anatomique selon la revendication 3, **caractérisée en ce que** le matériau est une mousse de polyuréthane et **en ce qu'**elle comporte des ondulations de forme conique à sommets arrondis dans toute partie avoisinant la peau de l'utilisateur.

7. Bio-coiffe anatomique selon l'une quelconque des revendications précédentes, **caractérisée en ce qu'**elle comprend un espace (D) prévu pour recevoir un détecteur de la position 'sur le dos' de l'utilisateur.

8. Bio-coiffe anatomique selon l'une quelconque des revendications précédentes, **caractérisée en ce qu'**elle comprend un arceau élastique et plastiquement non déformable (A) encastré dans une partie de la bio-coiffe anatomique correspondant au front et aux tempes de l'utilisateur cet arceau étant centré sur le milieu d'une partie de la bio-coiffe anatomique correspondant au front de l'utilisateur.

9. Bio-coiffe anatomique selon l'une quelconque des revendications précédentes, **caractérisée en ce qu'**elle comporte des cavités intérieures pour recevoir les oreilles de l'utilisateur sans exercer de pressions sur celles-ci lorsque l'utilisateur est en position debout.

10. Bio-coiffe anatomique selon la revendication 9 **caractérisée en ce que** lesdites cavités se prolongent vers l'avant pour que les sons extérieurs puissent être entendus.

11. Bio-coiffe anatomique selon la revendication 9 ou 10, **caractérisée en ce qu'**au moins une des cavités est conçue pour recevoir un haut-parleur miniaturisé et relié électriquement au détecteur de la position 'sur le dos', placé dans un espace (D) ménagé dans la bio-coiffe anatomique.

12. Ensemble comportant une bio-coiffe anatomique selon l'une quelconque des revendications précédentes, enveloppée dans une enveloppe-taie (T) en tissu lavable.

13. Ensemble selon la revendication 12 où la partie de l'enveloppe-taie venant en contact avec le front de l'utilisateur a une élasticité parallèlement au front de l'utilisateur permettant d'exercer une faible pression uniformement repartie et constante sur le front de l'utilisateur.

14. Ensemble, selon la revendication 12 ou 13, à l'enveloppe-taie duquel sont fixées par des points d'encrage définitif ou amovible :
- une mentonnière (M) simple ou double, reglable ou non en longueur et fabriquée en tissu lavable et non extensible ;
- des bandelettes (B) réglables ou non en longueur et fabriquées en tissu lavable et non extensible ; et
- une barette anti-ronflement (R) réglable en longueur et élasticité.

15. Utilisation d'une bio-coiffe anatomique ou d'un ensemble selon l'une quelconque des revendications précédentes à la place d'un oreiller ou d'un traversin.

## Patentansprüche

1. Anatomische Biohaube, die für eine ständige Verwendung an Stelle eines Kopfkissens und/oder einer Schlummerrolle durch natürliche Personen bestimmt ist, die dem Kopf ihres Benutzers während seines Schlafes und/oder seiner Ruhe in einer schlafenden/liegenden Position formmäßig nachgibt und mit diesem verbunden ist, wobei die anatomische Biohaube umfasst:
- einen vorderen Teil,
- zwei Teile, die den Stellen der Ohren entsprechen, und
- einen Teil, der dem Zentrum der Wölbung oder dem hinteren Bereich des Schädels entspricht,
und die anatomische Biohaube **dadurch gekennzeichnet ist, daß** unter Berücksichtigung der dem Benutzer eigenen körperlichen Ausbildungen
- die Dicke der anatomischen Biohaube in dem vorderen Teil derart beschaffen ist, daß, wenn sich der Benutzer in einer schlafenden/liegenden Position auf dem Bauch befindet, die anatomische Biohaube die Freigabe der Nase des Benutzers gewährleistet,
- die Dicken der anatomischen Biohaube in den Teilen, die den Stellen der Ohren entsprechen, derart beschaffen sind, daß, wenn sich ein Benutzer in schlafender/liegender Position auf der Seite befindet, die anatomische Biohaube die Einhaltung der Zervikallinie, erster Halswirbel, zweiter Halswirbel, Hinterhauptloch des Benutzers, gewährleistet,
- die Dicke der anatomischen Biohaube in dem Teil, der dem Zentrum der Wölbung des hinteren Schädels entspricht, derart beschaffen ist, daß, wenn sich der Benutzer in schlafender/liegender Position auf dem Rücken befindet, die anatomische Biohaube die Einhaltung der Zervikallinie erster Halswirbel, zweiter Halswirbel, Hinterhauptloch, des Benutzers gewährleistet,
- wobei die Teile miteinander verbunden sind, wobei die Verbindungen, die ihre Dicken betreffen, stufenweise erfolgen, um die geschlossene Kontur der anatomischen Biohaube, die praktisch rund ist, um den Kopf des Benutzers zu vollenden,
dank dessen die anatomische Biohaube auch gewährleistet:
die Einhaltung der Zervikallinie, erster Halswirbel, zweiter Halswirbel, Hinterhauptloch des Benutzers, wenn er sich in schlafenden/liegenden Zwischenpositionen zu den vorgenannten Positionen befindet, und gleichzeitig die Beseitigung jeder Möglichkeit des Kontakts des Gesichts des Benutzers mit jedem anderen Gegenstand, der schädliche Druckstellen hervorrufen kann, unabhängig von der schlafenden/liegenden Position des Benutzers auf einer Fläche frei von jedem Gegenstand während seines Schlafes und/oder seiner Ruhe.

2. Anatomische Biohaube nach Anspruch 1, **dadurch gekennzeichnet, daß** die anatomische Biohaube jede beliebige Form zwischen einer reduzierten Form gleich einem Turban und einer vollendeten Form einnehmen kann, die zur Gänze die Ohren und die Wölbung des hinteren Schädels bedeckt.

3. Anatomische Biohaube nach einem der Ansprüche 1 oder 2, **dadurch gekennzeichnet, daß** sie aus einem nicht starren und elastischen Material hergestellt ist.

4. Anatomische Biohaube nach einem der Ansprüche 1 oder 2, **dadurch gekennzeichnet, daß** sie von einer dichten, mit Luft aufblasbaren Hülle gebildet wird.

5. Anatomische Biohaube nach Anspruch 3, **dadurch gekennzeichnet, daß** das Material ein Polyurethanschaum oder ein Gummischwamm ist.

6. Anatomische Biohaube nach Anspruch 3, **dadurch gekennzeichnet, daß** das Material ein Polyurethanschaum ist und daß sie Wellungen konischer Form mit abgerundeten Spitzen in jedem Teil der an der Haut des Benutzers anliegt, umfasst.

7. Anatomische Biohaube nach einem der vorstehenden Ansprüche, **dadurch gekennzeichnet, daß** sie einen Raum (D) umfasst, der dazu vorgesehen ist, einen Sensor für die Position "auf dem Rücken" des Benutzers aufzunehmen.

8. Anatomische Biohaube nach einem der vorstehenden Ansprüche, **dadurch gekennzeichnet, daß** sie einen elastischen und plastisch nicht verformbaren Bogen (A) umfasst, der in einem Teil der anatomischen Biohaube eingesetzt ist, der der Stirn und den Schläfen des Benutzers entspricht, wobei dieser Bogen auf der Mitte eines Teils der anatomischen Biohaube zentriert ist, der der Stirn des Benutzers entspricht.

9. Anatomische Biohaube nach einem der vorstehenden Ansprüche, **dadurch gekennzeichnet, daß** sie innere Hohlräume umfasst, um die Ohren des Benutzers aufzunehmen, ohne Druck auf diese auszuüben, wenn sich der Benutzer in aufrechter Position befindet.

10. Anatomische Biohaube nach Anspruch 9, **dadurch gekennzeichnet, daß** sich die Hohlräume nach vorne verlängern, damit Laute von außen gehört werden können.

11. Anatomische Biohaube nach Anspruch 9 oder 10, **dadurch gekennzeichnet, daß** mindestens einer der Hohlräume derart ausgeführt ist, um einen Miniatur-Lautsprecher aufzunehmen, der elektrisch mit dem Sensor für die Position "auf dem Rücken" verbunden ist, der in einem Raum (D) vorgesehen ist, der in der anatomischen Biohaube ausgespart ist.

12. Einheit, umfassend eine anatomische Biohaube nach einem der vorstehenden Ansprüche, umgeben von einem Kissenbezug (T) aus einem waschbaren Stoff.

13. Einheit nach Anspruch 12, bei der der Teil des Kissenbezugs, der mit der Stirn des Benutzers in Kontakt kommt, eine Elastizität parallel zur Stirn des Benutzers aufweist, die es ermöglicht, einen geringen, gleichmäßig verteilten und konstanten Druck auf die Stirn des Benutzers auszuüben.

14. Einheit nach Anspruch 12 oder 13, deren Kissenbezug durch fixe oder abnehmbare Befestigungspunkte befestigt ist, **gekennzeichnet durch**:
- einen einfachen oder doppelten Kinnhalter (M), der in der Länge einstellbar ist oder nicht und aus einem nicht dehnbaren waschbaren Stoff hergestellt ist;
- in der Länge einstellbare oder nicht einstellbare Bänder (B), die aus einem nicht dehnbaren waschbaren Stoff hergestellt sind;
- und ein Steg gegen das Schnarchen (R), der in der Länge einstellbar und elastisch ist.

15. Verwendung einer anatomischen Biohaube oder einer Einheit nach einem der vorstehenden Ansprüche an Stelle eines Kopfkissens oder einer Schlummerrolle.

## Claims

1. ANATOMIC BIO-CAP destined to be used instead of a pillow and/or a bolster by physical persons, matching and fixedly fitting to an user's head during his sleep and/or his rest in lying down/streched position, ANATOMIC BIO-CAP comprising :
- a front part,
- two parts corresponding to the ears locations and
- a part corresponding to the center of the rear convexity of the skull,
ANATOMIC BIO-CAP **characterised in that**, owing to the user's particular physical conformations,
- the thickness of the ANATOMIC BIO-CAP in its front part is such that, when the user is lying in prone position, the ANATOMIC BIO-CAP ensures the relieving of the user's nose,
- the thickness of the ANATOMIC BIO-CAP in its parts corresponding to the ears places is such that, when the user is lying in one side positions, the ANATOMIC BIO-CAP ensures the respect of the user's line along cervices, axis, atlas, occipital cavity,
- the thickness of the ANATOMIC BIO-CAP in its part corresponding to the center of the rear convexity of the skull is such that when the user is lying in supine position, the ANATOMIC BIO-CAP ensures the respect of the user's line along cervices, axis, atlas, occipital cavity,
said parts being linked together, the thickness of the linking areas being gradual, to achieve the closed contour of the ANATOMIC BIO-CAP around the user's head, whereby the ANATOMIC BIO-CAP ensures also:
the respect of the user's line along cervices, axis, atlas, occipital cavity when he is lying in intermediate positions as regards to those mentioned and simultaneously, the elimination of any possibility of contacts of the user's face with any other objet which could create detrimental pressures, whatever the streched out/lying position of the user on a surface cleared of any objet, during his sleep and/or his rest.

2. ANATOMIC BIO-CAP according to claim 1, **characterised in that**, the ANATOMIC BIO-CAP can take any form between a reduced one similar to a turban and an accomplished one which covers in totality the ears and the rear skull convexity.

3. ANATOMIC BIO-CAP according to the claim 1 and/or 2, **characterised in that**, it is made of a non-rigid and elastic material.

4. ANATOMIC BIO-CAP according to claim 1 and/or 2, **characterised in that**, it is constituted by an air-tight inflatable envelope.

5. ANATOMIC BIO-CAP according to claim 3, **characterised in that** the material is a polyurethane foam or a porifera sponge.

6. ANATOMIC BIO-CAP according to claim 3, **characterised in that** the material is a polyurethane foam and it has cone-shaped undulations with rounded summits in every part neighbouring the user's skin.

7. ANATOMIC BIO-CAP according to any of precedent claims, **characterised in that**, it comprise a space (D) destined for receiving a detector of user's supine position.

8. ANATOMIC BIO-CAP according to any of precedent claims **characterised in that**, it contain an elastic but plastically non deformable ring bow (A) embedded in a portion of the ANATOMIC BIO-CAP corresponding to user's forehead and temples, said ring bow being centered on the middle of a part of the ANATOMIC BIO-CAP corresponding to the user's forehead.

9. ANATOMIC BIO-CAP according to any of precedent claims, **characterised in that** it comprise internal cavities to receive the user's ears without exerting pressures on them when the user is standing up.

10. ANATOMIC BIO-CAP according to claim 9 **characterised in that** said cavities are forwardly prolonged in order to allow the hearing of external sounds.

11. ANATOMIC BIO-CAP according to claim 9 or 10 **characterised in that**, at least one of the cavities is provided to receive an ear electrically connected to the detector for the supine position situated in a space (D) in the ANATOMIC BIO-CAP.

12. ENSEMBLE containing an ANATOMIC BIO-CAP according to any of the precedents claims, envelopped in an envelop-case (T) of washable woven.

13. ENSEMBLE according to claim 12 wherein the part of the envelop-case in contact with the user's forehead has an elasticity paralel to the user's forehead.

14. ENSEMBLE according to claims 12 or 13, to which are fixed to the envelop-case through permanent or detachable points :
- one simple or double chin-strap (M), adjustable or not adjustable in lenght and made of washable and non extensible woven;
- straps (B) adjustable or not adjustable in length and made of washable and non extensible woven ; and
- an anti-snoring strap (R) adjustable in length and elasticity.

15. The utilisation of an ANATOMIC BIO-CAP according to any of precedent claims, instead of a pilow or a bolster.
